# EUROPÄISCHE PATENTSCHRIFT

(11) **EP 3 289 905 B1**
(45) Veröffentlichungstag und Bekanntmachung des Hinweises auf die Patenterteilung: **26.06.2019**
(21) Anmeldenummer: 17186161.0
(22) Anmeldetag: 14.08.2017
(51) Int. Cl.: A42B 3/08, A42B 3/10, A61F 9/06, A42B 3/22

(54) **ARBEITSSCHUTZHELM MIT LICHTBOGENSCHUTZ**
INDUSTRIAL SAFETY HELMET WITH ARC PROTECTION
CASQUE DE PROTECTION POURVU D'UNE PROTECTION ANTI-ARC ÉLECTRIQUE

(30) Priorität: 01.09.2016 DE 202016005358 U
(43) Veröffentlichungstag der Anmeldung: 07.03.2018
(73) Patentinhaber: ENHA GmbH, 66620 Nonnweiler (DE)
(72) Erfinder: Engelhard, Jörg, 51503 Rösrath (DE)
(74) Vertreter: dompatent von Kreisler Selting Werner - Partnerschaft von Patent- und Rechtsanwälten mbB

(56) Entgegenhaltungen:
- WO-A1-2013/164520
- DE-A1-102014 004 558
- GB-A- 777 357

## Beschreibung

Die Erfindung betrifft einen Arbeitsschutzhelm mit einem Lichtbogenschutz.

Elektriker sind beim Schweißen den Gefahren eines Lichtbogens ausgesetzt. Zwar tragen Elektriker typischerweise Arbeitsschutzhelme mit einem schwenkbaren Visier, welches das Gesicht aus frontaler Blickrichtung bedeckt. Jedoch besteht insbesondere bei Lichtbögen der Klasse 2 7000 A und höher die Tendenz, dass der Lichtbogen das Visier unterläuft und am Kinn der den Helm tragenden Person Verbrennungen hervorruft. Die typischerweise getragene Schutzanzugsjacke ist zwar mit einem Kragen versehen. Jedoch verbleibt zwischen dem unteren Rand des Visiers und dem oberen Rand des Kragens ein kritischer Bereich. Dokument WO 2013/164520 A1 offenbart einen Schutzhelm mit einem am Visier angebrachten Kinnschutz.

Der Erfindung liegt die Aufgabe zugrunde, einen Arbeitsschutzhelm mit verbessertem Schutz gegen Lichtbögen zu schaffen

Der erfindungsgemäße Helm ist definiert durch die Merkmale von Anspruch 1.

Demnach ist an dem zur Befestigung am Kinn vorgesehenen Kinnriemen ein Schutz aus einem flexiblen Material derart vorgesehen, dass zumindest Bereiche des Kinns, der Wangen und der vorderen Halspartie von dem Schutz verdeckt werden. Das flexible Material ist an dem Kinnriemen aufgehängt und stützt sich am Kinn der den Helm tragenden Person ab. Der Schutz sollte so ausgebildet sein, dass er auf den Wangen und dem Kinn aufliegt und von dort ausreichend herabhängt, um auch die Halspartie zu schützen.

Zwischen den Befestigungspunkten des Schutzes an dem Kinnriemen im Bereich des Randes der Helmschale weist der Schutz einen ersten Rand auf, der zusammen mit einem vorderen Abschnitt des Helmschalenrandes eine erste Aussparung für Mund, Nase und Augen der den Helm tragenden Person bildet. Diese erste Aussparung kann von dem Schwenkvisier des Helms im verschwenkten Zustand verdeckt werden. Aus frontaler Ansicht sollten Visier und Schutz einander überlappen, um den verbleibenden Spalt möglichst gering zu halten und die Gefahr eines zwischen Schutz und Helmvisier hindurchtretenden Lichtbogens zu minimieren.

Zwischen den beiden Befestigungspunkten des Schutzes mit dem Kinnriemen im Bereich der einander gegenüberliegenden seitlichen Abschnitte des Helmschalenrandes weist der Schutz eine zweite Kante auf, die zusammen mit einem hinteren Abschnitt des Helmschalenrandes eine Aussparung für den Hinterkopf bildet.

Der Schutz kann aus einem im Wesentlichen viereckigen Material gebildet sein. Im Bereich zweier benachbarter Ecken des Schutzes ist dieser jeweils mit einem Abschnitt des Kinnriemens im Bereich des seitlichen Helmschalenrandes verbunden. Der Schutz kann hier mit dem Kinnriemen fest vernäht oder mit einer lösbaren Verbindung, wie zum Beispiel einem Klettverschluss, befestigt sein.

Im Bereich zweier weiterer Ecken kann der Schutz zum Verbinden dieser Ecken im Nackenbereich ausgebildet sein. Beispielsweise können die Ecken des Schutzes mit einem Klettverschluss oder einer anderen lösbaren Verbindung versehen sein.

Der Schutz sollte aus einem feuerfesten Stoffmaterial bestehen, das eine ausreichende Widerstandsfähigkeit gegen die Temperaturen von Lichtbögen aufweist.

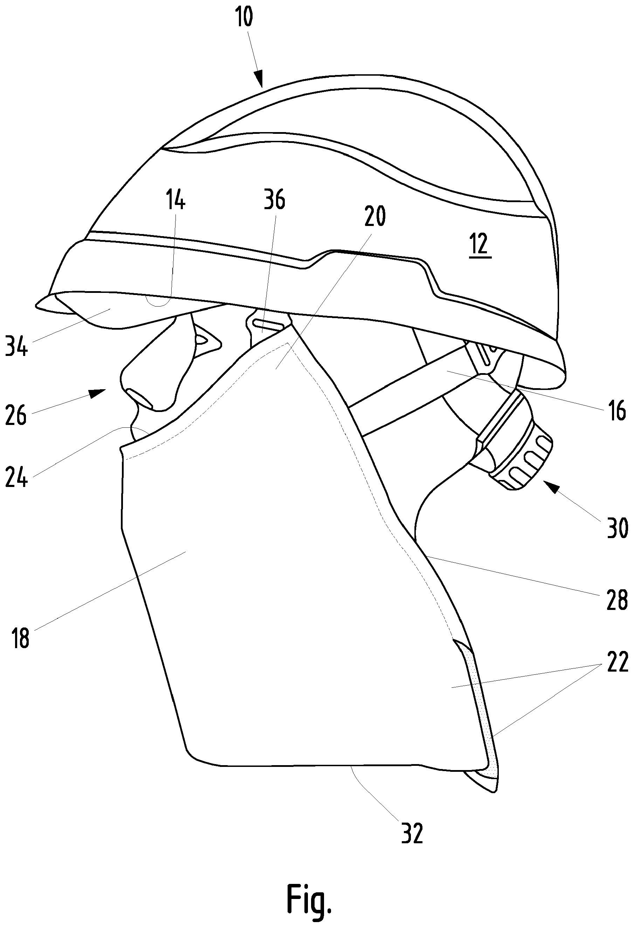

Im Folgenden wird anhand der Figur ein Ausführungsbeispiel der Erfindung näher erläutert. Die Figur zeigt eine seitliche Ansicht des Schutzhelms mit Lichtbogenschutz.

Der Schutzhelm 10 weist eine gewölbte Helmschale 12 auf, die mit einem unteren umlaufenden Rand 14 versehen ist. In einem vorderen Bereich des Randes 14 ist die Helmschale 12 mit einem schwenkbaren Visier 34 versehen. Die Figur zeigt das Visier im geöffneten Zustand, in dem das Gesicht der den Helm 10 tragenden Person nicht von dem Visier verdeckt wird.

Im Bereich einander gegenüberliegender seitlicher Abschnitte des Helmrandes 14 sind Enden 36 eines zur Befestigung am Kinn ausgebildeten Kinnriemens 16 mit der Helmschale 12 verbunden. Ein weiterer Abschnitt des Kinnriemens 16 ist im Bereich des hinteren Abschnitts des Helmrandes 14 mit der Helmschale 12 verbunden.

Der Schutz 18 besteht aus einem feuerfesten, flexiblen Stoffmaterial und weist im nicht am Helm befestigten, plan ausgebreiteten Zustand eine im Wesentlichen viereckige Form auf. Zwei benachbarte Ecken 20 sind jeweils im Bereich der Enden 36 mit dem Kinnriemen 16 verbunden. Vorteilhaft ist eine lösbare Verbindung über Druckknöpfe oder Klettverschlüsse, um den Schutz unabhängig von dem Helm reinigen zu können.

Zwischen den Ecken 20 ist der Schutz mit einer ersten, vorderen Kante 24 versehen, die zusammen mit einem vorderen Abschnitt des Helmschalenrandes 14 eine erste Aussparung 26 für Mund, Nase und Augen bildet. Diese Aussparung 26 kann zumindest aus frontaler Ansicht von dem Visier 34 im verschwenkten Zustand vollständig verdeckt werden. Die Kante 24 liegt auf den Wangen und dem Kinn der den Helm tragenden Person auf.

Die beiden übrigen Ecken 22 des Schutzes 18 sind mit zueinander komplementären Verbindungselementen zum lösbaren Verbinden der Ecken 22 aneinander, zum Beispiel mit einem Klettverschluss oder Druckknöpfen, versehen. Dadurch können die Ecken 22 im Nackenbereich der den Helm 10 tragenden Person aneinander befestigt werden, um den Schutz 18 um den Hals herum zu schließen und ein Verrutschen des Schutzes zu verhindern.

Dabei verläuft zwischen jeder der beiden Ecken 22 und jeder der beiden Ecken 20 eine zweite Kante 28 des Schutzes. Wenn die beiden Ecken 22, wie in der Figur dargestellt, miteinander verbunden sind, läuft die zweite, hintere Kante 28 vollständig von dem einen Ende 36 des Kinnriemens 16 zu dessen gegenüberliegendem Ende. Die zweite Kante 28 bildet zusammen mit einem hinteren Abschnitt des Helmschalenrandes 14 eine zweite, hintere Aussparung 30 für den Hinterkopf. Dadurch kann der Kopf auch mit geschlossenem Schutz 18 ausreichend bewegt werden.

Der Schutz 18 weist zudem eine dritte Kante 32 auf, die als unterer Rand, wie in der Figur dargestellt, zwischen den beiden Ecken 22 verläuft. Der Schutz 18 sollte derart ausgebildet sein, dass die untere Kante 18 auf den Schultern der den Helm tragenden Person aufliegt.

## Patentansprüche

1. Arbeitsschutzhelm (10) mit einer gewölbten Helmschale (12) mit einem umlaufenden Rand (14) und einem Kinnriemen (16) zur Befestigung am Kinn der den Helm (10) tragenden Person,
**dadurch gekennzeichnet,**
**dass** auf einander gegenüberliegenden Seiten des Kinnriemens (16) im Bereich des Randes (14) ein Schutz (18) aus einem flexiblen Material derart angebracht ist, dass zumindest Kinn-, Wangen- und Halsbereich der den Helm (10) tragenden Person vor Lichtbögen geschützt ist.

2. Arbeitsschutzhelm (10) nach Anspruch 1, **dadurch gekennzeichnet, dass** der Schutz aus einem im Wesentlichen viereckförmigen Material gebildet ist und im Bereich zweier benachbarter Ecken an dem Kinnriemen (16) befestigt ist.

3. Arbeitsschutzhelm (10) nach Anspruch 2, **dadurch gekennzeichnet, dass** der Schutz (18) im Bereich der beiden übrigen Ecken (22) zum Verbinden dieser Ecken im Nackenbereich der den Helm (10) tragenden Person ausgebildet ist.

4. Arbeitsschutzhelm (10) nach einem der vorhergehenden Ansprüche, **dadurch gekennzeichnet, dass** der Schutz (18) eine zwischen den beiden Befestigungspunkten mit dem Kinnriemen (16) verlaufende erste Kante (24) aufweist, die zusammen mit einem vorderen Abschnitt des Randes (14) der Helmschale (12) eine erste Aussparung (26) für Nase, Mund und Augen der den Helm (10) tragenden Person bildet.

5. Arbeitsschutzhelm (10) nach Anspruch 4, **dadurch gekennzeichnet, dass** der Helm (10) ein schwenkbares Visier (34) aufweist, das die erste Aussparung (26) im verschwenkten Zustand vollständig verdeckt.

6. Arbeitsschutzhelm (10) nach einem der vorhergehenden Ansprüche, **dadurch gekennzeichnet, dass** der Schutz (18) eine zwischen den beiden Befestigungspunkten mit dem Kinnriemen (16) verlaufende zweite Kante (28) aufweist, die zusammen mit einem hinteren Abschnitt des Randes (14) eine zweite Aussparung (30) für den Hinterkopf der den Helm (10) tragenden Person bildet.

7. Arbeitsschutzhelm (10) nach einem der vorhergehenden Ansprüche, **dadurch gekennzeichnet, dass** der Schutz (18) aus einem feuerfesten Stoffmaterial besteht.

8. Arbeitsschutzhelm (10) nach einem der vorhergehenden Ansprüche, **dadurch gekennzeichnet, dass** der Kinnriemen (16) auf einander gegenüberliegenden Seiten im Bereich seitlicher Abschnitte des Randes (14) an der Helmschale befestigt ist.

## Claims

1. An industrial safety helmet (10) having a curved helmet shell (12) with a circumferential brim (14), and a chin strap (16) for fastening to the chin of a person wearing said helmet (10),
**characterized in that**
on opposite sides of said chin strap (16) in the region of said brim (14) a guard (18) from a flexible material is attached such that at least chin, cheek and neck regions the person wearing said helmet (10) are protected against electric arcs.

2. The industrial safety helmet (10) according to claim 1, **characterized in that** said helmet is made from a substantially quadrangular material and is fastened to the chin strap (16) in the region of two adjacent corners.

3. The industrial safety helmet (10) according to claim 2, **characterized in that** in the region of the other two corners (22) the guard (18) is configured for connecting these corners in the nape region of the person wearing said helmet (10).

4. The industrial safety helmet (10) according to any one of the preceding claims, **characterized in that** the guard (18) comprises a first edge (24) extending between the two points of fastening to the chin strap (16), which edge, together with a front portion of the brim (14) of the helmet shell (12), defines a first recess (26) for nose, mouth and eyes of the person wearing said helmet (10).

5. The industrial safety helmet (10) according to claim 4, **characterized in that** said helmet (10) comprises a pivoted visor (34) which completely covers the first recess (26) in its pivoted state.

6. The industrial safety helmet (10) according to any one of the preceding claims, **characterized in that** the guard (18) comprises a second edge (28) extending between the two points of fastening to the chin strap (16), which edge, together with a rear portion of the brim (14), defines a second recess (30) for the back of the head of the person wearing said helmet (10).

7. The industrial safety helmet (10) according to any one of the preceding claims, **characterized in that** the guard (18) is made from a fireproof fabric material.

8. The industrial safety helmet (10) according to any one of the preceding claims, **characterized in that** the chin strap (16) is fastened to the helmet shell on opposite sides in the region of lateral portions of the brim (14).

## Revendications

1. Casque de sécurité (10) comportant une coque de casque (12) bombée et pourvue d'un bord périphérique (14) et d'une mentonnière (16) destinée à être fixée au menton de la personne portant le casque (10),
**caractérisé en ce que**
une protection (18) en matériau souple est montée sur des côtés opposés de la mentonnière (16), dans la région du bord (14) de manière à protéger au moins la région du menton, des joues et du cou de la personne portant le casque (10) contre des arcs électriques.

2. Casque de sécurité (10) selon la revendication 1, **caractérisé en ce que** la protection est formée d'un matériau sensiblement quadrangulaire et est fixée à la mentonnière (16) dans la région de deux coins adjacents.

3. Casque de sécurité (10) selon la revendication 2, **caractérisé en ce que** la protection (18) est conçue dans la région des deux autres coins (22) pour relier ces coins dans la région du cou de la personne portant le casque (10) .

4. Casque de sécurité (10) selon l'une des revendications précédentes, **caractérisé en ce que** la protection (18) comporte un premier bord (24) qui s'étend entre les deux points de fixation à la mentonnière (16) et qui forme, conjointement avec une partie avant du bord (14) de la coque de casque (12), un premier évidement (26) destiné au nez, à la bouche et au yeux de la personne portant le casque (10).

5. Casque de sécurité (10) selon la revendication 4, **caractérisé en ce que** le casque (10) comporte une visière pivotante (34) qui couvre complètement le premier évidement (26) à l'état pivoté.

6. Casque de sécurité (10) selon l'une des revendications précédentes, **caractérisé en ce que** la protection (18) comporte un deuxième bord (28) qui s'étend entre les deux points de fixation à la mentonnière (16) et qui forme, conjointement à une partie arrière du bord (14), un deuxième évidement (30) destiné à l'arrière de la tête de la personne portant le casque (10).

7. Casque de sécurité (10) selon l'une des revendications précédentes, **caractérisé en ce que** la protection (18) comprend un matériau réfractaire.

8. Casque de sécurité (10) selon l'une des revendications précédentes, **caractérisé en ce que** la mentonnière (16) est fixée à la coque de casque sur des côtés opposés au niveau des zones latérales du bord (14).
